# EUROPEAN PATENT SPECIFICATION

(11) **EP 0 312 246 B1**
(45) Date of publication and mention of the grant of the patent: **30.11.1994**
(21) Application number: 88309273.6
(22) Date of filing: 05.10.1988
(51) Int. Cl.: C07G 17/00, A61K 35/44, A01N 1/02, C12Q 1/02

(54) **Inhibitor of angiogenesis**
Angiogeneseinhibitor
Inhibiteur d'angiogenèse

(30) Priority: 13.10.1987 US 107936
(43) Date of publication of application: 19.04.1989
(73) Proprietor: The Children's Medical Center Corporation, Boston, Massachusetts 02115 (US)
(72) Inventor: Folkman, Moses Judah, Brookline, MA 02146 (US); Doctrow, Susan R., Roslindale, MA 02130 (US)
(74) Representative: Moon, Donald Keith

(56) References cited:
- WO-A-86/04783
- AT-B- 310 350
- US-A- 4 534 967
- ARCHIVES OF OPHTALMOLOGY, vol. 103, December 1985; B.M. GLASER et al., pp. 1870-1875#

## Description

This invention relates to newly discovered angiogenesis inhibitors, more particularly to an essentially purified inhibitor of capillary endothelial cell growth and angiogenesis obtained from corneas, preferably from mammalian corneas. In its most preferred aspects, the invention relates to such an inhibitor obtainable by water extraction at 4°C from corneal tissue, e.g. the outer layer, including the corneal epithelium and Bowman's basement membrane, of bovine corneas. Although the inhibitor is specifically obtainable from the outer cornea, it appears to be dispersed throughout the whole cornea, and can be obtained from whole cornea extracts. The inventicn also relates to a method of purifying such inhibitors to angiogenesis. Finally, the invention relates to methods of preserving corneal tissue.

Inhibitors of angiogenesis are useful inter alia in the treatment of pathologic conditions involving or dependent upon the occurrence of neovascularization, such as chronic inflammation, certain immune responses, and neoplasia (including most solid tumors). Other inhibitors of angiogenesis have previously been described, such as combinations of heparin or certain heparin fragments with certain steroids as taught by Crum et.al., Science, Vol. 230, 1375-1378 (1976). Other known angiogenesis inhibitors include protamine and platelet factor 4. See generally, M.J. Folkman, "Tumor Angiogenesis" Adv. Canc. Res. 43: 175 and Ciba Symp. However, such prior inhibitors in some cases have exhibited undesired biological side effects or have lacked specificity and high efficacy.

The inhibitor of the present invention, because it can be isolated from biological sources such as mammalian corneas, is relatively free from undesirable side effects and may be particularly effective in treatment of mammals displaying ocular angiogenesis such as corneal neovascularization, as well as in treatment of tumor angiogenesis or the angiogenesis associated with arthritis and psoriasis. It may be combined with a suitable non-toxic physiologically acceptable vehicle or carrier for administration topically, as in eyedrops, or systemically as in intramuscular, intravascular, or intraperitoneal injection. It may also be administered with previously described inhibitors of angiogenesis, enabling the latter to be used at lower, less toxic doses. Finally, the inhibitor may be included in an aqueous saline solution used to store corneal tissue. The effective dosage varies over a considerable range depending upon the condition being treated, the weight of the patient, and the mode of administration, but those skilled in the art can readily determine the dosage in any particular case by simple experiment. In many cases, the dosage would generally be in the range from 0.1 to 50 mg/Kg body weight.

The inhibitor of the present invention preferably has at least one or more of the following characteristics:
(a) it is somewhat heat stabile, e.g., it retains significant inhibitory activity after heating for five minutes at 100°C;
(b) it can be extracted in large volumes of chloroform (e.g., over 50 ml chloroform per lyophilized inhibitor from 100 corneas);
(c) it is of relatively low molecular weight, e.g., it passes through a molecular sieve which prevents passage of molecules larger than 2000 daltons;
(d) it is eluted from chloroform-equilibrated silica gel HPLC column with chloroform-methanol at about 10-15% v/v methanol;
(e) it is eluted from a reverse phase water-equilibrated n-octadecyl-modified silica gel column with methanol-water at approximately 80% methanol by volume.

The angiogenesis inhibitors of the present invention are preferably obtained by extraction of corneas, preferably mammalian corneas, such as bovine, porcine, rabbit or human corneas, and preferably from the outer corneal layer, including the corneal epithelium and Bowman's basement layer. The solvent used for the extraction is preferably water, although the inhibitors also have some solubility in methanol, chloroform and acetonitrile. Preferably the extraction is carried out at low temperatures, e.g., around 1°C to 10°C, and most preferably at about 1°C.

The extracted inhibitor is then purified, preferably by HPLC and/or reverse phase HPLC techniques, such as those mentioned above. Preferably the inhibitor is purified to at least 90% by weight, more preferably to at least 95%.

A convenient assay or test for the presence of the angiogenesis inhibitor of the present invention has also been discovered, in that the effect of the inhibitor on endothelial cells, namely rounding of the cells (without detachment) and inhibition of their growth, is easily observed.

The following specific example is intended to illustrate more clearly the nature of the invention and not to act as a limitation upon its scope. Unless otherwise indicated herein, all percentages are on a weight basis, and temperatures are in degrees centigrade, uncorrected.

### Examples

The outer layer of bovine corneas, approximately 20% of the total corneal thickness and including the corneal epithelium and Bowman's basement membrane as determined histologically, was dissected away from the corneas on ice. The dissected layers from 30 corneas were suspended in about 15 ml distilled water enclosed in a cellophane dialysis bag serving as a molecular sieve which prevents passage of molecules larger than 3500 daltons. The bag was placed in a 50 ml sterile plastic tube and the tube was filled with distilled water and capped. The tube was incubated at 4°C for at least 3 hours, preferably 18-24 hr. with gentle rotation. The solution outside of the dialysis bag was then collected, transferred to a glass flask and lyophilized. The dry powder, which could be stored at -20°C, was then extracted twice with 5 ml chloroform, the undissolved residue was removed by centrifugation, and the extract was concentrated about 3.5-fold by evaporation at reduced pressure (Savant Speed-Vac apparatus). This procedure was repeated several times with successive specimens of corneal layers.

Forty-six ml of the above extract (made from a total of 129 corneas) was concentrated to about 13 ml and filtered through a 0.45 micron Nylon 66 syringe filter. It was applied to a semi-preparative HPLC silica column (Supelco Supelcosil, 5 micron particles; 25 cm length; 10 mm diameter equilibrated in chloroform) at a flow rate of 2 ml/min, and the column was washed until the absorbance at 280 nm returned to the baseline. The column was then eluted with 180 ml solvent having a linear gradient from 100% chloroform to 50% methanol/50% chloroform by volume and collected in 3 ml fractions. The solvent was evaporated from the fractions at room temperature and each residue dissolved in 200 µl distilled water.

A 20 µl aliquot of each redissolved fraction was subjected to a preliminary in vitro test for inhibitory activity by the following procedure. Approximately 5000 capillary endothelial cells were plated in a 96-well tissue culture plate with Eagle's medium and 10% serum. The plates could be used for the assay within 24 hours and remained useful for 7-8 days. The 20 µl specimen of each fraction was air dried, redissolved in 60-100 µl of the tissue culture medium, and transferred to a well of the plate. Presence of inhibitor was demonstrated by the rounding (but not detachment) of the endothelial cells as well as inhibition of their growth and was readily detected by visual observation; in the case of fractions containing no inhibitor no rounding occurred, the cells remaining flat and their growth uninhibited.

By the foregoing test the inhibitor was found to be present in the two fractions eluting at about 15% methanol. The two fractions, which contained the active inhibitor after being dissolved in water were pooled and a 50 µl sample assayed for activity as an inhibitor by the generally accepted CAM procedure as described in Crum et al., loc. cit., the sample first being divided into two equal aliquots, air dried, and each dissolved in 10 µl of 0.45% solution of methyl cellulose in distilled water at 4°C which was then air dried on a 2 mm. diameter Teflon mold. The resulting discs were each implanted on 6-day old chorioallantoic membranes and after 48 hours each exhibited an avascular zone greater than 4 mm. in diameter, demonstrating inhibitory effect of the sample on angiogenesis in vivo.

A further specimen of pooled eluted active fractions dissolved in water, obtained from 195 bovine corneas as described above, was subjected to reverse phase high performance liquid chromatography by injecting it onto an analytical C₁₈ HPLC column (silica gel modified by reaction with n-octadecyl trichlorosilane, Supelco, Supelcosil LC-18, 5 micron particles, 15 cm x 4.6 mm) and equilibrating with water; the column was washed with water, then eluted with 30 ml solvent having a linear gradient of 100% water to 100% methanol at a flow rate of 1 ml/min., and fractions of one ml each were collected. The solvent was removed from each fraction by evaporation at reduced pressure and the residue dissolved in 50µl water. The inhibitor was found, by the endothelial cell rounding test described above, to be in the fraction eluting at approximately 80% methanol/20% water by volume.

A specimen of the original water extract from the bovine corneas before lyophilization was boiled for five minutes and was then assayed by the in vitro cell-rounding test and found to retain its inhibitory activity.

In a subsequent experiment, corneas were suspended in distilled water and dialyzed, generally as described above, and the solution was lyophilized. The resulting dry powder was extracted in a large excess of chloroform. For example the lyophylized extract from 154 corneas was extracted with 100 ml. chloroform. The inhibitor activity, purified by this additional chloroform extraction and silica chromatography, retained substantial activity after boiling.

A specimen of the original water extract (before chloroform extraction) was incubated with an inscluble pronase preparation (Streptomyces griseus; Sigma) at 0.1 mg (0.0115 units)-/ml in PBS (pH 7.0) for 10 hr. at 37°C. The protease was removed by centrifugation at 14,000 x g. The pronase-treated extract induced endothelial cell rounding with similar potency to that of untreated extract.

Other methods for extracting and purifying the newly discovered corneal angiogenesis inhibitors will be readily apparent to the skilled in the art from the above disclosure. While the above example is directed to extraction of the inhibitor from bovine corneas, the same or similar techniques can be used to obtain angiogenesis inhibitor from other corneas, preferably mammalian corneas, such as human, rabbit, porcine, equine, canine, feline, or other corneas. Other tests for antiangiogenesis activity, such as those referred to in the references cited above, can also be used. The above-described endothelial cell assay can also be used to advantage for assaying for antiangiogenesis activity of other angiogenesis inhibitors.

In subsequent experiments conducted generally as described above using a molecular seive that will prevent passage of molecules larger than 2000 daltons, the inhibitor passes through this seive.

Finally, the inhibiitor serves to preserve corneal tissue which is being transported or stored for transplantation. Specifically, undesirable vasculation has been associated with increased risk of tissue rejection. The usual aqueous storage medium for such tissue depletes inhibitor in the tissue.

By including inhibitor in the storage medium, such depletion, and therefore the risk of tissue rejection by the host, can be diminished. Specifically, those skilled in the art are aware of standard aqueous saline cornea storage media, to which effective concentrations of inhibitor are added to reduce depletion of the inhibitor from corneal tissue. Significantly, the practice of preserving corneal tissue in ice also depletes the inhibitor, as compared to preserving the tissue in dry ice.

## Claims

1. An essentially purified inhibitor of capillary endothelial cell growth obtainable from corneal tissue by extraction by the following method:
a) providing said corneal tissue;
b) extracting said tissue with water to produce an aqueous extract;
c) removing from said extract molecules of a molecular weight greater than a molecular mass on the order of 2000-3500 daltons;
d) removing the water from said extract to produce a residue;
e) extracting said residue with chloroform to produce a chloroform solution;
f) applying said chloroform solution to a chloroform equilibrated silica gel HPLC column; and
g) eluting said column with a chloroform-methanol mixture.

2. An inhibitor according to claim 1 wherein said corneal tissue includes corneal epithelium and Bowman's basement membrane.

3. An inhibitor according to claim 1 or 2, wherein the inhibitor retains at least a portion of its inhibitory activity after heating for five minutes at 100°C.

4. An inhibitor according to claim 3, wherein the inhibitor retains at least a portion of its inhibitory activity after incubation for 10 hours at 37°C and pH 7.0 in phosphate buffered saline containing 0.1 mg/ml of protease.

5. An inhibitor obtained by the method according to claim 1, wherein said method comprises passing a solution comprising said inhibitor through a molecular sieve which prevents passage of molecules larger than 2000 daltons, and said inhibitor being contained in filtrate which passes through said sieve.

6. An inhibitor according to any of the preceding claims wherein said chloroform methanol solution is 10-15% (by volume) methanol.

7. An inhibitor according to any of the preceding claims, wherein said method comprises passing a solution comprising said inhibitor over a reverse phase water-equilibrated n-octodecyl- modified silica gel column and eluting said inhibior from said column with a methanol-water mixture.

8. An inhibitor according to any of the preceding claims, wherein said method comprises extracting said tissue with water at about 1°C to 10°C.

9. A method of producing an inhibitor of capillary endothelial cell growth from corneal tissue by:
a) providing said corneal tissue;
b) extracting said tissue with water to produce an aqueous extract;
c) removing from said extract molecules of a molecular weight greater than a molecular mass on the order of 2000-3500 daltons;
d) removing the water from said extract to produce a residue;
e) extracting said residue with chloroform to produce a chloroform solution;
f) applying said chloroform solution to a chloroform equilibrated silica gel HPLC column; and
g) eluting said column with a chloroform-methanol mixture.

10. A method according to claim 9 wherein said corneal tissue includes corneal epithelium and Bowman's basement membrane.

11. A method according to claim 9 or 10 wherein said method comprises passing a solution comprising said inhibitor through a molecular sieve which prevents passage of molecules larger than 2000 daltons, and said inhibitor being contained in filtrate which passes through said sieve.

12. A method according to any one of claims 9 to 11 wherein said chloroform methanol solution is 10-15% (by volume) methanol.

13. A method according to any one of claims 9 to 12 wherein said method comprises passing a solution comprising said inhibitor over a reverse phase water-equilibrated n-octodecyl- modified silica gel column and eluting said inhibitor from said column with a methanol-water mixture.

14. A method according to any one of claims 9 to 13 wherein said method comprises extracting said tissue with water at about 1°C to 10°C.

15. A pharmaceutical composition, comprising the angiogenesis inhibitor of claim 1, and a pharmaceutically acceptable carrier.

16. A method of assaying for the presence of an angiogenesis inhibitor according to claim 1 comprising exposing endothelial cells to a material to be tested and determining whether the endothelial cells become rounded.

## Patentansprüche

1. Im wesentlichen gereinigter Inhibitor gegen kapillares Endothelwachstum, wobei der Inhibitor gemäß dem folgenden Verfahren durch Extraktion aus Hornhautgewebe erhalten wird:
a) es wird das Hornhautgewebe bereitgestellt;
b) es wird das Gewebe mittels Wasser extrahiert, um einen wässrigen Extrakt herzustellen;
c) aus dem Extrakt werden alle Moleküle mit einem Molekulargewicht größer als eine Molekularmasse in der Größenordnung von 2000 bis 3500 Dalton entfernt;
d) von dem Extrakt wird das Wasser entfernt, um einen Rest herzustellen;
e) der Rest wird mit Chloroform extrahiert, um eine Chloroformlösung zu erzeugen;
f) die Chloroformlösung wird in eine Chloroform-äquilibrierte Silikagel-HPLC-Säule gegeben; und
g) die Säule wird mit einer Chloroform-Methanol-Mischung eluiert.

2. Inhibitor nach Anspruch 1, bei dem das Hornhautgewebe Hornhautepithel und die Bowman's Basismembrane umfaßt.

3. Inhibitor nach Anspruch 1 oder 2, der nach einer 5-minütigen Erwärmung auf 100° C wenigstens einen Teil seiner Inhibitoraktivität behält.

4. Inhibitor nach Anspruch 3, der nach einer 10-stündigen Inkubation bei 37° C in einer phosphat-gepufferten physiolgischen Kochsalzlösung mit einem pH-Wert von 7,0, die 0,1 mg/ml Protease enthält, wenigstens einen Teil seiner inhibierenden Eigenschaft behält.

5. Inhibitor, der nach dem Verfahren gemäß Anspruch 1 erhalten wurde, wobei das Verfahren das Passieren einer den Inhibitor enthaltenden Lösung durch ein Molkularsieb umfaßt, das einen Durchgang von Molekülen größer als 2000 Dalton verhindert, und wobei der Inhibitor in dem Filtrat enthalten ist, das durch das Sieb hindurch gelangt.

6. Inhibitor nach einem der vorhergehenden Ansprüche, wobei die Chloroform-Methanol-Lösung 10%-15% Methanol (bezogen auf das Volumen) enthält.

7. Inhibitor nach einem der vorhergehenden Ansprüche, wobei das Verfahren das Passieren der den Inhibitor enthaltenen Lösung durch eine Wasser-äquilibrierte und mit n-Octodecyl modifizierte Silikagelsäule mit umgekehrten Phasen umfaßt und wobei das Verfahren das Eluieren des Inhibitors aus der Säule mit einer Methanol-Wasser-Lösung beinhaltet.

8. Inhibitor nach einem der vorhergehenden Ansprüche, wobei das Verfahren das Extrahieren des Gewebes mit Wasser bei etwa 1° C bis 10° C beinhaltet.

9. Verfahren zum Herstellen eines Inhibitors gegen kapillars Endothelwachstum aus Hornhautgewebe, indem
a) das Hornhautgewebe bereitgestellt wird;
b) das Gewebe mittels Wasser extrahiert wird, um einen wässrigen Extrakt herzustellen;
c) aus dem Extrakt alle Moleküle mit einem Molekulargewicht größer als eine Molekularmasse in der Größenordnung von 2000 bis 3500 Dalton entfernt werden;
d) von dem Extrakt das Wasser entfernt wird, um einen Rest herzustellen;
e) der Rest mit Chloroform extrahiert wird, um eine Chloroformlösung zu erzeugen;
f) die Chloroformlösung in eine Chloroform- äquilibrierte Silikagel-HPLC-Säule gegeben wird; und
g) die Säule mit einer Chloroform-Methanol-Mischung eluiert wird.

10. Verfahren nach Anspruch 9, bei dem das Hornhautgewebe Hornhautepithel und die Bowman's Basismembrane umfaßt.

11. Verfahren Anspruch 9 oder 10, das das Passieren einer den Inhibitor enthaltenden Lösung durch ein Molkularsieb umfaßt, das einen Durchgang von Molekülen größer als 2000 Dalton verhindert, und bei dem der Inhibitor in dem Filtrat enthalten ist, das durch das Sieb hindurch gelangt.

12. Verfahren nach einem der Ansprüche 9 bis 11, bei dem die Chloroform-Methanol-Lösung 10%-15% Methanol (bezogen auf das Volumen) enthält.

13. Verfahren nach einem der Ansprüche 9 bis 12, das das Passieren der den Inhibitor enthaltenen Lösung durch eine Wasser-äquilibrierte und mit n-Octodecyl modifizierte Silikagelsäule mit umgekehrten Phasen umfaßt und bei dem der Inhibitor aus der Säule mit einer Methanol-Wasser-Lösung eluiert wird.

14. Verfahren nach einem der Ansprüche 9 bis 13, bei dem das Gewebe mit Wasser bei etwa 1° C bis 10° C extrahiert wird.

15. Pharmazeutische Zusammensetzung, die den Angiogeneseinhibitor nach Anspruch 1 und einen pharmazeutisch verträglichen Träger aufweist.

16. Verfahren, um das Vorhandensein eines Angiogeneseinhibitors nach Anspruch 1 zu testen, wobei Endothelzellen dem zu untersuchenden Material ausgesetzt werden und bestimmt wird, ob die Endothelzellen sich verrunden.

## Revendications

1. Inhibiteur de la croissance de cellules endothéliales de capillaires, sous forme pratiquement purifiée obtenu à partir du tissu de la cornée par extraction selon le procédé suivant consistant à :
(a) disposer dudit tissu de la cornée;
(b) extraire dudit tissu de l'eau pour produire un extrait aqueux;
(c) éliminer dudit extrait les molécules d'un poids moléculaire supérieur à une masse moléculaire de l'ordre de 2000-3500 daltons;
(d) éliminer l'eau dudit extrait pour obtenir un résidu;
(e) extraire ledit résidu avec du chloroforme pour produire une solution à base de chloroforme;
(f) appliquer ladite solution à base de chloroforme sur une colonne HPLC de gel de silice équilibré avec du chloroforme et
(g) éluer ladite colonne avec un mélange chloroforme-méthanol.

2. Inhibiteur selon la revendication 1 dans lequel ledit tissu de la cornée comprend l'épithélium de la cornée et la membrane basale de Bowman.

3. Inhibiteur selon la revendication 1 ou 2, dans lequel l'inhibiteur conserve au moins une partie de son activité inhibitrice après avoir été chauffé pendant 5 minutes à 100°C.

4. Inhibiteur selon la revendication 3, dans lequel l'inhibiteur conserve au moins une partie de son activité inhibitrice après incubation pendant 10 heures à 37° C et à un pH de 7 dans un tampon phosphate salin contenant 0,1 mg/ml de protéase.

5. Inhibiteur obtenu selon le procédé de la revendication 1, dans lequel ledit procédé comprend le passage de la solution comprenant ledit inhibiteur à travers un tamis moléculaire empêchant le passage des molécules d'une taille supérieur à 2000 daltons et ledit inhibiteur étant contenu dans le filtrat qui passe à travers ledit tamis.

6. Inhibiteur selon l'une quelconque des revendications précédentes, dans lequel ladite solution de chloroforme-méthanol contient de 10 à 15 % (en volume) de méthanol.

7. Inhibiteur selon l'une quelconque des revendications précédentes, dans lequel ledit procédé comprend les étapes consistant à chromatographier en phase inverse une solution comprenant ledit inhibiteur sur une colonne de gel de silice modifiée par des groupes n-octodécyl- et équilibrée par de l'eau et éluer ledit inhibiteur de ladite colonne avec un mélange méthanol-eau.

8. Inhibiteur selon l'une quelconque des revendications précédentes, caractérisé en ce que ledit procédé comprend l'extraction dudit tissu avec de l'eau à environ 1° C jusqu'à 10° C.

9. Procédé pour la production d'un inhibiteur de la croissance de cellules endothéliales de capillaires provenant de tissu de la cornée consistant à :
(a) disposer dudit tissu de la cornée;
(b) extraire dudit tissu de l'eau pour produire un extrait aqueux;
(c) éliminer dudit extrait les molécules d'un poids moléculaire supérieur à une masse moléculaire de l'ordre de 2000-3500 daltons;
(d) éliminer l'eau dudit extrait pour obtenir un résidu;
(e) extraire ledit résidu avec du chloroforme pour produire une solution à base de chloroforme;
(f) appliquer ladite solution à base de chloroforme sur une colonne HPLC de gel de silice équilibrée avec du chloroforme et
(g) éluer ladite colonne avec un mélange chloroforme-méthanol.

10. Procédé selon la revendication 9, dans lequel ledit tissu de la cornée comprend l'épithélium de la cornée et la membrane basale de Bowman.

11. Procédé selon la revendication 9 ou 10, dans lequel ledit procédé comprend le passage d'une solution comprenant ledit inhibiteur à travers un tamis moléculaire qui empêche le passage des molécules d'une taille supérieure à 2000 daltons et ledit inhibiteur étant contenu dans le filtrat qui passe à travers ledit tamis.

12. Procédé selon l'une quelconque des revendications 9 à 11, dans lequel ladite solution chloroforme-méthanol contient de 10 à 15 % (en volume) de méthanol.

13. Procédé selon l'une quelconque des revendications 9 à 12, dans lequel ledit procédé comprend la chromatographie en phase inverse d'une solution comprenant ledit inhibiteur sur une colonne de gel de silice modifiée par des groupes n-octodécyl- équilibrée par de l'eau et l'élution dudit inhibiteur de ladite colonne à l'aide d'une mélange méthanol-eau.

14. Procédé selon l'une quelconque des revendications 9 à 13, dans lequel ledit procédé comprend l'extraction dudit tissu avec de l'eau à environ 1° C jusqu'à 10° C.

15. Composition pharmaceutique comprenant l'inhibiteur de l'angiogénèse de la revendication 1 et un véhicule pharmaceutiquement acceptable.

16. Procédé pour la détection d'un inhibiteur de l'angiogénèse selon la revendication 1 comprenant les étapes consistant à exposer les cellules endothéliales au matériau devant être testé et à déterminer si les cellules endothéliales prennent une forme arrondie.
